# EUROPÄISCHE PATENTSCHRIFT

(11) **EP 1 190 699 B1**
(45) Veröffentlichungstag und Bekanntmachung des Hinweises auf die Patenterteilung: **29.11.2006**
(21) Anmeldenummer: 01119285.3
(22) Anmeldetag: 10.08.2001
(51) Int. Cl.: A61K 8/86, A61Q 5/06

(54) **Haarstylingstift auf Basis von Polyethylenglykolen verschiedener Molekulargewichte**
Hair styling stick based on polyethylene glycols of different molecular weights
Bâton de coiffure à base de polyéthylène glycols de poids moléculaires différents

(30) Priorität: 22.09.2000 DE 10047038
(43) Veröffentlichungstag der Anmeldung: 27.03.2002
(73) Patentinhaber: Wella Aktiengesellschaft, 64274 Darmstadt (DE)
(72) Erfinder: Birkel, Susanne, 95496 Glashütten (DE); Wendel, Harald, 64372 Ober-Ramstadt (DE); Franzke, Michael, 64380 Rossdorf (DE); Stein, Bernd, 63768 Hösbach (DE)

(56) Entgegenhaltungen:
- EP-A- 0 865 786
- GB-A- 1 563 824
- US-A- 4 938 954
- US-A- 5 690 924

## Beschreibung

Gegenstand der Erfindung ist ein Haarstylingstift (Styling Stick) mit einer Zusammensetzung von fester, wachsartiger Konsistenz enthaltend eine Kombination eines hochmolekularen Polyethylenglykols mit einem niedriger molekularen Polyethylenglykol.

Stylingwachs-Zusammensetzungen sind bekannte Produkte zur Haarbehandlung. Sie finden insbesondere Anwendung, um kurzes bis mittellanges Haar trendgerecht in Form zu bringen. Mit derartigen Produkten sind beispielsweise Frisurengestaltungen in Form von hochgestellten Ponypartien, gestriegelten Vorderköpfen, sowie sogenannte "Dirt-Look"- oder "Spiky Look"-Frisuren möglich. Auch lassen sich hiermit Konturen und Texture in der Frisur erzeugen. Herkömmliche Stylingwachs-Produkte werden üblicherweise in Tiegeln angeboten. Die Entnahme der Produktmasse erfolgt mit den Fingern. Das Wachs wird in den Handflächen verteilt, wobei es aufgrund der über die Hände übertragenen Körperwärme erweicht. Durch die Erweichung wird die Einarbeitung des ansonsten zu festen Wachses in das Haar ermöglicht. Nach Einarbeitung in das Haar kühlt das Wachs wieder ab, erhärtet dabei und die gestaltete Frisur erhält Stabilität und einen leichten Wet-Look.

Der Nachteil dieser Applikationsform ist, dass die Hände des Anwenders intensiv mit dem Wachs in Berührung kommen und anschließend gereinigt werden müssen. Die Aufgabe der vorliegenden Erfindung bestand darin, ein Produkt zu entwickeln, welches im wesentlichen die Produkteigenschaften von Tiegel-Wachsen aufweist, d.h. dem Haar Glanz, Pflege und Halt verleiht, dabei aber in einer anderen Applikationsform angewandt wird, bei der die Finger bzw. die Handflächen nicht benutzt werden müssen. Eine geeignete Applikationsform ist ein Haarstylingstift, im folgenden Styling Stick genannt. An die hierfür benötigte Zusammensetzung sind eine Reihe von Anforderungen zu stellen. Die Zusammensetzung darf nicht zu hart sein und muss sich auch ohne durch Erwärmung hervorgerufene Erweichung bei direkter Auftragung auf das Haar gut auf dem Haar verteilen lassen. Gleichzeitig darf die Zusammmensetzung nicht zu weich sein, da ansonsten der gewünschte Stand und Halt der erstellten Frisur nicht erreicht wird. Der Abrieb darf nicht zu gering und nicht zu stark sein und die Konsistenz darf nicht spröde sein. Ausserdem darf die Zusammensetzung nicht zu klebrig sein, da ansonsten Haare an der Stiftmasse haften bleiben, wenn der Stift bei der Anwendung über das Haar gestrichen wird.

Herkömmliche Stylingsticks sind in der Regel auf der Basis von hydrophoben Wachsen, Fetten und Ölen aufgebaut. Sie enthalten einen großen Anteil an hydrophoben Stoffen wie z.B. pflanzlichen oder tierischen Wachsen, Fettsäureestern, Fettalkoholen etc.. Derartige Produkte haben den Nachteil, dass sie schlecht aus dem Haar auswaschbar sind und eine relativ hohe Belastung des Haares bewirken.

Aus der EP 0 301 197 A1 ist ein Haarwachs bekannt, welches eine Kombination von hochmolekularem Polyethylenglykol (MG = 3000 bis 5000), oxethyliertem und hydriertem Rizinusöl und niedrigmolekularem Polyethylenglykol (MG = 100 bis 300) enthält. Es werden nur Tiegel-Produkte beschrieben, aber keine Sticks. Mit der in der EP 0 301 197 A1 beschriebenen Kombination von zwei Polyethylenglykolen stark unterschiedlichen Molekulargewichts ist die Herstellung von Haarstylingsticks mit völlig zufriedenstellenden Produkteigenschaften nicht möglich. Bei stark unterschiedlichem Molekulargewicht weisen die Sticks eine zu grobe und spröde Konsistenz auf.

Aus der US 5 690 924 ist ein Haarwachs bekannt, welches eine Kombination von hochmolekularem Polyethylenglykol (MG=3000) und niedrigmolekularem Polyethylenglykol (MG=600) enthält.

Aus Janistyn, Handbuch der Kosmetika und Riechstoffe, Band 3 (1973) Seite 325 ist eine Stangenpomade bestehend aus 60 Gew.% Polyethylenglykol 4000 und 40 Gew.% Ethylhexandiol bekannt. Mit einer derartig zusammengesetzten Stangenpomade behandelte Haare besitzen jedoch weder einen befriedigenden Glanz noch eine ausreichende Formstabilität oder ein befriedigendes Standvermögen. Zudem handelt es sich bei Ethylhexandiol um eine reizende Substanz, deren Einsatz in kosmetischen Produkten nach Möglichkeit vermieden werden sollte.

Es wurde nun gefunden, dass die Anforderungen erfüllt werden durch einen Haarstylingstift (Styling Stick) mit einer Zusammensetzung von fester, wachsartiger Konsistenz enthaltend eine Kombination eines hochmolekularen Polyethylenglykols mit einem niedriger molekularen Polyethylenglykol eines bestimmten Molekulargewichts.

Gegenstand der Erfindung ist daher ein Haarstylingstift mit einer festen, wachsartigen Zusammensetzung enthaltend
(A) mindestens ein hochmolekulares Polyethylenglykol mit einem Molekulargewicht von 2500 bis 5000 und
(B) mindestens ein niedriger molekulares Polyethylenglykol mit einem Molekulargewicht von 370 bis 800 sowie gegebenenfalls Wasser, ein- oder mehrwertige C1-C5-Alkohole, Emulgatoren, Parfüm, Farbstoffe und/oder Perlglanzpigmente.

Die erfindungsgemäße Kombination von Polyethylenglykolen mit ganz bestimmten Molekulargewichten ist hervorragend dazu geeignet, um Haarstylingprodukte in Stiftform herzustellen. Das hochmolekulare Polyethylenglykol (A) ist bei Raumtemperatur (20-25°C) wachsartig fest. Es wird in Konzentrationen von vorzugsweise 30 bis 55 Gew.%, besonders bevorzugt von 35 bis 45 Gew.% eingesetzt. Das Molekulargewicht beträgt 2500 bis 5000 g/mol, vorzugsweise 2700 bis 3500 g/mol. Polyethylenglykole besitzen die allgemeine Formel H(OCH₂CH₂)ₙOH. Geeignete hochmolekulare Polyethylenglykole sind solche mit n = 57 bis 113, vorzugsweise mit n = 61 bis 79. Geeignete Polyethylenglykole haben die INCI-Bezeichnungen PEG-60 (n = 60), PEG-75 (n = 75), PEG-90 (n = 90) und PEG-100 (n = 100). Besonders bevorzugt sind PEG-60 und PEG-75. Handelsprodukte weisen in der Regel eine Molekulargewichtsverteilung auf. Geeignete Handelsprodukte sind z.B. Polyglykol 3000 mit einem Molekulargewicht von 2700 bis 3000 oder Polyglykol 4000 der Firma Clariant mit einem Molekulargewicht von 3700 bis 4500. Hierbei ist das Polyglykol 3000 vorteilhafter als das Polyglykol 4000, da es in der erfindungsgemäßen Kombination zu weicheren Sticks mit vermehrtem Abrieb führt.

Das niedrigermolekulare Polyethylenglykol (B) ist bei Raumtemperatur (20-25°C) zumindest weichwachsartig oder vorzugsweise flüssig. Es wird in Konzentrationen von vorzugsweise 40 bis 70 Gew.%, besonders bevorzugt von 52 bis 60 Gew.% eingesetzt. Das Molekulargewicht beträgt 370 bis 800 g/mol, vorzugsweise 400 bis 640 g/mol. Geeignete niedrigmolekulare Polyethylenglykole sind solche der Formel H(OCH₂CH₂)ₙOH mit n = 8 bis 16, vorzugsweise mit n = 9 bis 14. Geeignete Polyethylenglykole haben die INCI-Bezeichnungen PEG-8 (n = 8), PEG-9 (n = 9), PEG-10 (n = 10), PEG-12 (n = 12), PEG-14 (n = 14) und PEG-16 (n = 16). Besonders bevorzugt sind PEG-9, PEG-10, PEG-12 und PEG-14. Geeignete Handelsprodukte sind z.B. Polyglykol 400 mit einem Molekulargewicht von 380 bis 420 oder Polyglykol 600 der Firma Clariant mit einem Molekulargewicht von 570 bis 630. Hierbei hat sich das Polyglykol 600 als vorteilhafter als das Polyglykol 400 erwiesen.

Es hat sich gezeigt, dass die Stylingsticks eine schlechtere, gröbere und sprödere Konsistenz aufweisen, je weiter das Molekulargewicht der beiden Polyethylenglykole auseinanderliegt. Ein Vergleich einer erfindungsgemäßen Stick-Zusammensetzung mit einer Zusammensetzung entsprechend der EP 0 301 197, welche als niedrigmolekulares Polyethylenglykol ein Polyglykol 200 (molare Masse 190 bis 210 g/mol) enthielt, führte zu einem wesentlich gröberen und spröderen Stick. Diese beobachtete Sprödigkeit hängt vermutlich mit dem Kristallisationsverhalten der Gesamtzusammensetzung zusammen. Die Herstellung des Sticks erfolgt in der Regel in der Weise, dass die Komponenten bei einer solchen erhöhten Temperatur vermischt werden, bei der auch das hochmolekulare Polyethylenglykol flüssig vorliegt. Anschließend wird abgekühlt, was in der Regel mittels einer zusätzlichen äußeren Kühlung erfolgt. Hierbei erstarrt das hochmolekulare Polyethylenglykol, wobei die Kristallisation exotherm verläuft. Es hat sich gezeigt, dass die Auswahl des niedrigmolekularen Polyethylenglykols einen Einfluss auf das Kristallisationsverhalten hat. Je weiter die Molekulargewichte der eingesetzten Polyethylenglykole auseinanderliegen, umso gröber und spröder wird der Stick.

Andererseits soll das niedrigmolekulare Polyethylenglykol bei Raumtemperatur noch flüssig sein, da sonst die Produktleistungen hinsichtlich Glanz und Verteilbarkeit auf dem Haar beeinträchtigt sind. Sehr gut geeignet ist daher Polyglykol 600 mit einer molaren Masse von 570 bis 630 g/mol und einer Erstarrungstemperatur von 17 bis 22 °C, d.h. knapp unterhalb von Raumtemperatur.

Das Massenverhältnis von hochmolekularem Polyethylenglykol (A) zu niedrigmolekularem Polyethylenglykol (B) beträgt vorzugsweise von 1 : 0,9 bis 1 : 1,8, besonders bevorzugt von 1 : 1 bis 1 : 1,6.

Der erfindungsgemäße Stylingstick ermöglicht aufgrund der wachsartigen Konsistenz und seiner kohäsiven Eigenschaften eine individuelle Frisurengestaltung sowie die gezielte Behandlung einzelner Haarsträhnen. Die Produktmasse ist leicht auf dem Haar verteilbar, ohne dass die Hände oder Finger mit der Produktmasse in Berührung kommen. Das behandelte Haar zeichnet sich durch einen ausgeprägten Glanz sowie durch eine hohe Formstabilität der erstellten Frisur aus. Das Haar wird dabei nicht übermäßig belastet und die Produktmasse ist leicht auswaschbar.

Zusätzlich zu den vorstehend genannten Inhaltsstoffen kann die Stick-Zusammensetzung weitere Zusatzstoffe enthalten:
- Lösungsmittel wie Wasser oder ein- oder mehrwertige C1- bis C4-Alkohole, insbesondere Ethanol, Propanol, Glycerine oder Glykole in einer Menge bis zu 10 Gew.%, vorzugsweise 0,1 bis 8 Gew.%
- Kosmetische Farbstoffe in einer Menge bis zu 6 Gew.%, vorzugsweise 0,1 bis 4 Gew.%, z.B. C.I. Pigment Red 4 (C.I. 12 085), C.I. Pigment Green (C.I. 74 260), und/oder C.I. Vat Blue 4 (C.I. 69 800)
- Perlglanzpigmente in einer Menge von bis zu 25 Gew.%, vorzugsweise 1 bis 20 Gew.%, z.B. solche auf Titandioxid/Mica-Basis
- Emulgatoren in einer Menge von bis zu 25 Gew.%, vorzugsweise 0,1 bis 18 Gew.%, z.B. hydriertes und ethoxyliertes Rizinusöl
- Parfüm- und Duftstoffe in einer Menge von bis zu 2 Gew.%, vorzugsweise 0,01 bis 1 Gew.%
- Konservierungsmittel in einer Menge von bis zu 1 Gew.%, vorzugsweise 0,01 bis 0,5 Gew.%, insbesondere Parahydroxybenzoesäureester, Benzoesäure, Salicylsäure, Sorbinsäure, Mandelsäure, Polyhexamethylenbiguanid-hydrochlorid oder Isothiazolinonderivate, von denen sich die Sorbinsäure als besonders geeignet erwiesen hat
- Hydrophobe Wachse, Fette oder Öle, z.B. pflanzliche, tierische oder mineralische Wachse wie Ozokerit, Fettsäureester, Fettalkohole in einer Menge von bis zu 5 Gew.%, vorzugsweise 0,1 bis 4 Gew.%
- Filmbildende Polymere wie z.B. Polyvinylpyrrolidon oder Vinylpyrrolidon/Vinylacetat Copolymere in einer Menge von bis zu 5 Gew.%, vorzugsweise 0,1 bis 4 Gew.%
- Haarpflegende Zusätze wie z.B. Betain in einer Menge von bis zu 5 Gew.%, vorzugsweise 0,01 bis 4 Gew.%.

Die Herstellung der Stylingsticks erfolgt, indem die Komponenten bei einer erhöhten Temperatur, bei der das hochmolekulare Polyethylenglykol in flüssiger Form vorliegt (z.B. ca. 65 bis 90°C), unter Rühren aufgeschmolzen und miteinander vermischt werden. Anschließend wird die homogene Schmelze gegebenenfalls etwas abgekühlt und in noch flüssiger Form in Verpackungen mit der gewünschten Stick-Form (Stickhülsen) abgefüllt. Unter weiterer Abkühlung, vorzugsweise mit externer Kühlung (z.B. 0-10°C) erstarrt die Produktmasse. Bei schneller Abkühlung mit zusätzlicher externer Kühlung erhalten die Sticks eine größere Festigkeit als bei langsamerer Abkühlung ohne externe Kühlung.

Als Verpackungsmaterial können die üblichen, für kosmetische Sticks verwendeten Materialien eingesetzt werden, insbesondere Kunststoffe oder Metalle. Der geometrischen Form der Sticks sind keine Grenzen gesetzt. Es kann sich um eine zylindrische Form mit runder oder ovale Grundfläche aber auch um Formen mit eckigen Grundflächen handeln.

Die Applikation des Wachses in Stick-Form ist sehr einfach. Der Stift wird über das Haar gezogen und hinterläßt so die Produktmasse. Das Haar erhält dadurch Glanz, Texture und/oder Halt.

Die nachfolgenden Beispiele sollen den Gegenstand der Erfindung näher erläutern.

### Beispiele

Die Sticks mit den nachfolgenden Zusammensetzungen werden hergestellt, indem die Komponenten bei 85°C unter Rühren und Aufschmelzen vermischt werden. Nach Abkühlen auf 65-70°C werden die Massen in Stickhülsen abgefüllt. Unter Kühlung (4°C) erstarren die Massen in den Hülsen.

### Beispiel 1 Styling Stick

| | |
|---|---|
| 57,0 g | PEG-12 (Polyglykol 600) |
| 43,0 g | PEG-60 (Polyglykol 3000) |

### Beispiel 2 Styling Stick

| | |
|---|---|
| 56,1 g | PEG-12 (Polyglykol 600) |
| 43,0 g | PEG-60 (Polyglykol 3000) |
| 0,5 g | Wasser |
| 0,2 g | Parfüm |
| 0,1 g | Betain |
| 0,1 g | Glycerin |

### Beispiel 3 Styling Stick

| | |
|---|---|
| 58,7 g | PEG-12 (Polyglykol 600) |
| 40,0 g | PEG-60 (Polyglykol 3000) |
| 0,5 g | Wasser |
| 0,3 g | PEG-25 Hydrogenated Castor Oil |
| 0,3 g | Parfüm |
| 0,1 g | Betain |
| 0,1 g | Ozokerit |

### Beispiel 4 Styling Stick

| | |
|---|---|
| 55,45 g | PEG-12 (Polyglykol 600) |
| 43,00 g | PEG-60 (Polyglykol 3000) |
| 0,50 g | Wasser |
| 0,30 g | PEG-25 Hydrogenated Castor Oil |
| 0,30 g | Parfüm |
| 0,25 g | Sorbinsäure |
| 0,10 g | Betain |
| 0,10 g | Ozokerit |

## Patentansprüche

1. Haarstylingstift mit einer festen, wachsartigen Zusammensetzung enthaltend
(A) mindestens ein hochmolekulares Polyethylenglykol mit einem Molekulargewicht von 2500 bis 5000 und
(B) mindestens ein niedrigermolekulares Polyethylenglykol mit einem Molekulargewicht von 370 bis 800.

2. Haarstylingstift nach Anspruch 1, **dadurch gekennzeichnet, dass** das niedrigermolekulare Polyethylenglykol (B) bei Raumtemperatur (20-25°C) flüssig ist.

3. Haarstylingstift nach einem der vorhergehenden Ansprüche, **dadurch gekennzeichnet, dass** das hochmolekulare Polyethylenglykol (A) in einer Menge von 30 bis 55 Gew.% vorliegt.

4. Haarstylingstift nach einem der vorhergehenden Ansprüche, **dadurch gekennzeichnet, dass** das niedrigermolekulare Polyethylenglykol (B) in einer Menge von 40 bis 70 Gew.% vorliegt.

5. Haarstylingstift nach einem der vorhergehenden Ansprüche, **dadurch gekennzeichnet, dass** das Mengenverhältnis der Polyethylenglykole (A) : (B) von 1 : 0,9 bis 1 : 1,8 beträgt.

6. Haarstylingstift nach einem der vorhergehenden Ansprüche, **dadurch gekennzeichnet, dass** das hochmolekulare Polyethylenglykol (A) ein Molekulargewicht im Bereich von 2700 bis 3500 g/mol aufweist.

7. Haarstylingstift nach einem der vorhergehenden Ansprüche, **dadurch gekennzeichnet, dass** das niedrigermolekulare Polyethylenglykol (B) ein Molekulargewicht im Bereich von 400 bis 640 g/mol aufweist.

8. Haarstylingstift nach einem der vorhergehenden Ansprüche, **dadurch gekennzeichnet, dass** mindestens ein weiterer Zusatzstoff enthalten ist, ausgewählt aus Wasser, ein- oder mehrwertigen C1- bis C4-Alkoholen, kosmetischen Farbstoffen, Perlglanzpigmenten, Emulgatoren, Parfüm- und Duftstoffen, Konservierungsmitteln, hydrophoben Wachsen, Fetten und Ölen, filmbildenden Polymeren und haarpflegenden Zusätzen.

## Claims

1. Hairstyling stick with a solid, wax-like composition comprising
(A) at least one high molecular weight polyethylene glycol with a molecular weight of from 2500 to 5000 and
(B) at least one lower molecular weight polyethylene glycol with a molecular weight of from 370 to 800.

2. Hairstyling stick according to Claim 1, **characterized in that** the lower molecular weight polyethylene glycol (B) is liquid at room temperature (20-25°C).

3. Hairstyling stick according to one of the preceding claims, **characterized in that** the high molecular weight polyethylene glycol (A) is present in an amount of from 30 to 55% by weight.

4. Hairstyling stick according to one of the preceding claims, **characterized in that** the lower molecular weight polyethylene glycol (B) is present in an amount of from 40 to 70% by weight.

5. Hairstyling stick according to one of the preceding claims, **characterized in that** the quantitative ratio of the polyethylene glycols (A):(B) is from 1:0.9 to 1:1.8.

6. Hairstyling stick according to one of the preceding claims, **characterized in that** the high molecular weight polyethylene glycol (A) has a molecular weight in the range from 2700 to 3500 g/mol.

7. Hairstyling stick according to one of the preceding claims, **characterized in that** the lower molecular weight polyethylene glycol (B) has a molecular weight in the range from 400 to 640 g/mol.

8. Hairstyling stick according to one of the preceding claims, **characterized in that** at least one further additive is present, chosen from water, mono- or polyhydric C1- to C4-alcohols, cosmetic dyes, pearlescent pigments, emulsifiers, perfumes and fragrances, preservatives, hydrophobic waxes, fats and oils, film-forming polymers and haircare additives.

## Revendications

1. Bâton coiffant comportant une composition cireuse solide contenant
(A) au moins un polyéthylèneglycol de masse moléculaire élevée, ayant une masse moléculaire de 2 500 à 5 000 et
(B) au moins un polyéthylèneglycol de faible masse moléculaire, ayant une masse moléculaire de 370 à 800.

2. Bâton coiffant selon la revendication 1, **caractérisé en ce que** le polyéthylèneglycol (B) de faible masse moléculaire est liquide à la température ambiante (20-25 °C).

3. Bâton coiffant selon l'une quelconque des revendications précédentes, **caractérisé en ce que** le polyéthylèneglycol (A) de masse moléculaire élevée est présent en une quantité de 30 à 55 % en poids.

4. Bâton coiffant selon l'une quelconque des revendications précédentes, **caractérisé en ce que** le polyéthylèneglycol (B) de faible masse moléculaire est présent en une quantité de 40 à 70 % en poids.

5. Bâton coiffant selon l'une quelconque des revendications précédentes, **caractérisé en ce que** le rapport des quantités des polyéthylèneglycols (A):(B) va de 1:0,9 à 1:1,8.

6. Bâton coiffant selon l'une quelconque des revendications précédentes, **caractérisé en ce que** le polyéthylèneglycol (A) de masse moléculaire élevée a une masse moléculaire dans la plage de 2 700 à 3 500 g/mole.

7. Bâton coiffant selon l'une quelconque des revendications précédentes, **caractérisé en ce que** le polyéthylèneglycol (B) de faible masse moléculaire a une masse moléculaire dans la plage de 400 à 640 g/mole.

8. Bâton coiffant selon l'une quelconque des revendications précédentes, **caractérisé en ce qu'**est contenu au moins un autre additif choisi parmi l'eau, des alcools en C₁-C₄ mono- ou plurivalents, des colorants cosmétiques, des pigments nacrés, des émulsifiants, des parfums et des substances odoriférantes, des conservateurs, des cires, graisses et huiles hydrophobes, des polymères filmogènes et des additifs de soin capillaire.
